# EUROPÄISCHE PATENTSCHRIFT

(11) **EP 3 745 971 B1**
(45) Veröffentlichungstag und Bekanntmachung des Hinweises auf die Patenterteilung: **23.02.2022**
(21) Anmeldenummer: 19700567.1
(22) Anmeldetag: 09.01.2019
(51) Int. Cl.: A61B 17/70, A61B 17/86, A61B 90/00

(54) **POLYAXIALSCHRAUBE**
POLYAXIAL SCREW
VIS POLYAXIALE

(30) Priorität: 31.01.2018 DE 102018102173
(43) Veröffentlichungstag der Anmeldung: 09.12.2020
(73) Patentinhaber: Silony Medical International AG, 8500 Frauenfeld (CH)
(72) Erfinder: HEUER, Frank, 70794 Filderstadt (DE); MEHREN, Christoph, 81925 Muenchen (DE); HEMPFING, Axel, 34128 Kassel (DE); BENNEKER, Lorin M., 3037 HERRENSCHWANDEN (CH)
(74) Vertreter: DREISS Patentanwälte PartG mbB
(86) Internationale Anmeldenummer: PCT/EP2019/050420
(87) Internationale Veröffentlichungsnummer: WO 2019/149483

(56) Entgegenhaltungen:
- EP-A1- 2 502 594
- EP-A1- 2 586 392
- EP-A2- 2 638 874
- EP-B1- 2 502 594
- EP-B1- 2 586 392
- WO-A1-2015/155702

## Beschreibung

Die Erfindung betrifft eine Polyaxialschraube, insbesondere Pedikelschraube, mit einem einen Gewindeschaft und einen Kopf aufweisenden Schraubanker und mit einem in einer Seitenansicht U-förmigen und eine Aufnahmeöffnung für ein Korrekturelement, insbesondere einen Korrekturstab, aufweisenden Gabelkopf mit zwei Schenkeln, wobei der Gabelkopf eine axiale Richtung, eine hierzu radiale Richtung und eine Umfangsrichtung aufweist sowie ein distales Ende benachbart zu dem Schraubanker und ein hiervon in axialer Richtung abgewandtes proximales Ende aufweist, so dass auch eine distale Richtung und eine proximale Richtung definiert ist, wobei der Kopf des Schraubankers im eingesetzten Zustand in einem distalen Endbereich des Gabelkopfs polyaxial verschwenkbar gelagert ist und der Gabelkopf in einer vom Chirurgen intendierten Schwenkstellung bezüglich des Kopfs des im Knochen festgelegten oder festlegbaren Schraubankers fixierbar ist, wobei sich die Schenkel ausgehend von einem distalen Bereich des Gabelkopfs in proximaler Richtung erstrecken und proximal frei enden und zwischen sich die Aufnahmeöffnung für das Korrekturelement begrenzen, wobei die Schenkel einen radial äußeren Umfangsbereich aufweisen, in dem zum Ergreifen des Gabelkopfs mittels eines Handhabungsinstruments wenigstens eine Haltenut oder eine sonstige Instrumentenansetzstelle ausgebildet ist, und mit einem im Gabelkopf zwischen dem Kopf des Schraubankers und dem Korrekturelement anordenbaren Druckstück, das einerseits auf dem Kopf des Schraubankers aufsitzt und andererseits durch das Korrekturelement belastbar ist, wobei auf das Druckstück mittels des am Gabelkopf angreifenden Handhabungsinstruments oder mittels eines anderen Stellmittels eine temporär wirkende Kraft ausübbar ist, so dass der Gabelkopf hierdurch gegenüber dem Kopf des Schraubankers temporär in einer vom Chirurgen gewünschten Schwenkstellung unverstellbar gehalten ist, solange bis der Gabelkopf und der Schraubanker durch ein anderes Stellmittel dauerhaft gegeneinander fixiert werden, wobei das Druckstück im Bereich eines Schenkels des Gabelkopfs über ein Auflager gegen diesen Schenkel in axialer Richtung abgestützt ist und wobei das Druckstück diametral gegenüberliegend einen Aufnahmebereich für eine in axialer Richtung wirkende Stellkraft aufweist, welche das Druckstück in distaler Richtung bezüglich des Auflagers zu verschwenken sucht und hierdurch die temporär wirkende Kraft in Richtung auf den Kopf des Schraubankers bewirkt, wobei der Gabelkopf eine in axialer Richtung erstreckte Zugangsausnehmung bei dem anderen Schenkel aufweist, durch welche Zugang zu dem Aufnahmebereich des Druckstücks mittels des Handhabungsinstruments oder des anderen Stellmittels genommen werden kann, wobei dieser Schenkel des Gabelkopfs eine von innen in radialer Richtung nach außen erstreckte Ausnehmung aufweist, in welche das Druckstück in seiner bestimmungsgemäßen Montageposition mit seinem Aufnahmebereich von innen eingreift.

Eine derartige Polyaxialschraube ist Gegenstand der nicht vorveröffentlichten DE 10 2016 114 266.2 der Anmelderin. Eine weitere derartige Polyaxialschraube mit einem geringfügig schwenkbar bezüglich eines Auflagers vorgesehenen Druckstück zur Erzielung einer temporären Klemmung von Gabelkopf und Schraubanker ist auch bekannt aus WO 2015/155702 A1. Gemäß diesem Stand der Technik wird der Schraubanker wie auch das Druckstück durch eine sehr ausladende seitliche Öffnung in seine Montageposition gebracht. Diese seitliche Öffnung durchbricht den Gabelkopf naturgemäß von seinem distalen Ende her und stellt eine erhebliche Schwächung des Gabelkopfs dar.

Ausgehend von diesem Stand der Technik liegt der vorliegenden Erfindung die Aufgabe zugrunde, den Gabelkopf insgesamt stabiler auszubilden und weniger Bauraum für Füge- bzw. Montagevorgänge vorzuhalten.

Diese Aufgabe wird ausgehend von der eingangs genannten Polyaxialschraube erfindungsgemäß dadurch gelöst, dass der Gabelkopf im Bereich seines distalen Endes eine in der Umfangsrichtung durchgehend begrenzte Durchstecköffnung für den Schraubanker aufweist, so dass der Schraubanker in der axialen Richtung durch diese Durchstecköffnung hindurch in den Gabelkopf einsetzbar ist, und dass das Druckstück radial gegenüberliegend von seinem Aufnahmebereich in einem distalen und dem Kopf des Schraubankers zugewandten Endbereich eine derart ausgebildete randseitige Freischneidung aufweist, dass das Druckstück in schräg geneigtem Zustand mit seinem Aufnahmebereich voraus in der axialen Richtung von oben in den Gabelkopf einsetzbar und in seine bestimmungsgemäße Montageposition einschwenkbar ist, in der das Druckstück mit seinem Aufnahmebereich in die nach radial außen erstreckte Ausnehmung eingreift und auf dem Kopf des Schraubankers aufsitzt.

Dadurch, dass der Schraubanker durch die in Umfangsrichtung geschlossene Durchstecköffnung im Gabelkopf gefügt wird, braucht keine ausladende Queröffnung im Gabelkopf ausgebildet zu werden, die eine Reduzierung der Stabilität des Gabelkopfs bedeuten würde. Nach einem weiteren Erfindungsgedanken wird das Druckstück mit der vorstehend genannten Freischneidung so ausgebildet, dass es von oben in schräg geneigtem Zustand in den Gabelkopf eingeführt werden kann. Es muss also nicht wie bei der vorstehend genannten WO 2015/155702 A1 durch eine ausladende seitliche Einstecköffnung radial eingeführt werden. Die Freischneidung hat also den Sinn, dass das Druckstück beim Einschwenken in seine bestimmungsgemäße Montageposition an dem Schenkel innen vorbeikommt, also nicht mit einem distalen zylindrischen Rand kollidiert und am Einschwenken gehindert wird. Erst diese Ausbildung macht die Montage des Druckstücks in der beanspruchten Weise möglich, und es wurde festgestellt, dass hierdurch die temporäre Klemmung von Gabelkopf, Druckstück und Schraubanker nicht nachteilig beeinflusst wird. Gemäß der nicht vorveröffentlichten DE 10 2016 114 266.2 kann das Druckstück zwar auch axial gefügt werden; es ist dabei aber nicht schräg geneigt, sondern wird parallel zu seiner schlussendlichen Montageposition in den Gabelkopf eingesetzt. Dabei gleitet der radial vorstehende Aufnahmebereich für die temporäre Stellkraft entweder in einer radial innen ausgebildeten Zugangsöffnung bei einem der Schenkel, oder das Druckstück wird um 90° verdreht axial gefügt, so dass der Aufnahmebereich innerhalb der durch die Schenkel begrenzten U-Form nach unten gleitet und dann durch eine Rückdrehung des Druckstücks in einer radial innen ausgebildeten Umfangsnut in einem der Schenkel in die Montageposition gebracht wird. Sowohl diese radial innen ausgebildete Umfangsnut als auch die vorstehend erwähnte radial innen an einem Schenkel ausgebildete axiale Zugangsöffnung stellen jedoch eine Schwächung des betreffenden Schenkels dar. Auf beides kann bei der vorliegenden erfindungsgemäßen Weiterbildung der Polyaxialpedikelschraube verzichtet werden. Es braucht lediglich die eine von innen in radialer Richtung nach außen erstreckte Ausnehmung für den radial vorstehenden Aufnahmebereich des Druckstücks vorgesehen zu werden. Diese Ausnehmung muss lediglich etwas größer als der radial vorstehende Aufnahmebereich des Druckstücks ausgebildet werden, damit das Druckstück wie vorstehend beschrieben beim axialen Fügen in die bestimmungsgemäße Montageposition eingeschwenkt werden kann.

In Weiterbildung der erfindungsgemäßen Lösung erweist es sich als besonders vorteilhaft, wenn die beiden Schenkel des Gabelkopfs einen insbesondere konzentrischen Innenraum begrenzen und das Druckstück außerhalb seines Aufnahmebereichs für die Stellkraft und außerhalb der Freischneidung mit einem dem Innenraum entsprechenden insbesondere konzentrischen Umfang ausgebildet ist. Dies bedeutet nämlich, dass das Druckstück durch den Innenraum des Gabelkopfs auch axial geführt ist. Es kann dabei auch geringfügig spielbehaftet im Innenraum angeordnet sein, so dass es die Verschwenkung gegenüber dem Auflager im Zuge der temporären Fixierung ausführen kann. Das Druckstück ist also mit Ausnahme seines radial vorstehenden Aufnahmebereichs für die temporäre Klemmkraft wie in einem Topf im Gabelkopf aufgenommen und der inneren Formgebung dieses Topfs angepasst.

Es erweist sich als vorteilhaft, wenn die Freischneidung nur gerade derart bemessen ist, dass das Druckstück beim axialen Einfügen in die schlussendliche Montageposition eingeschwenkt werden kann. Hierfür erweist es sich als vorteilhaft, wenn die Freischneidung von einer nach außen konvex gewölbten Fläche oder von facettenartigen Flächensegmenten begrenzt ist. Diese facettenartigen Flächensegmente können dabei auch jeweils für sich genommen eben ausgebildet sein, jedoch sich derart aneinander anschließen, dass eine approximiert gewölbte Begrenzungsfläche entsteht.

Es kann sich herstellungstechnisch als vorteilhaft erweisen, wenn die von innen nach radial außen erstreckte Ausnehmung, in welche der radial vorstehende Aufnahmebereich des Druckstücks eingreift, durch den Schenkel des Gabelkopfs hindurchgeht und am Außenumfang des Schenkels mündet. Hierdurch lässt sich der durch den radial vorstehenden Aufnahmebereich des Druckstücks gebildete Hebelarm zur Erzeugung der temporären Klemmung verlängern. Eine praxiserhebliche Reduzierung der Stabilität kann gänzlich ausgeschlossen werden, wenn sich die Öffnung von radial innen nach radial außen hin verjüngt. In Weiterbildung dieses Gedankens erweist es sich als besonders vorteilhaft, wenn die von innen nach radial außen erstreckte Ausnehmung von einer distalen Flanke begrenzt ist, die von innen nach außen in proximaler Richtung geneigt ist. Insbesondere kann eine die Ausnehmung begrenzende proximale Flanke orthogonal zur axialen Richtung ausgebildet sein. Durch die Neigung der distalen Flanke ergibt sich eine Vergrößerung des Schwenkraums für den radial vorstehenden Aufnahmebereich des Druckstücks beim Einschwenken in die Montageposition erreichen.

Es erweist sich als ganz besonders vorteilhaft, wenn der nach radial außen erstreckte Aufnahmebereich des Druckstücks radial innen in axialer Richtung dicker ausgebildet ist als radial weiter außen, oder anders ausgedrückt wenn er sich nach radial außen verjüngt. Durch die Materialverstärkung weiter innen wird der Hebel des Druckstücks bei Ausübung der temporären Stellkraft gestärkt, und die eingeleitete Stellkraft wird wirksamer in das Druckstück eingeleitet und in eine axiale Klemmkraft auf den Kopf des Schraubankers umgewandelt.

Es erweist sich auch als vorteilhaft, wenn der nach radial außen erstreckte Aufnahmebereich proximal eine orthogonal zur Längsrichtung verlaufende Flanke und distal eine schräg zur Längsrichtung geneigte Flanke aufweist.

Die eingangs erwähnte in der axialen Richtung erstreckte Zugangsausnehmung zu dem Aufnahmebereich des Druckstücks ist vorzugsweise als in ihrer Umfangsrichtung durchgehend begrenzte axiale Öffnung ausgebildet. Auf diese Weise kann sie eine Führungsfunktion für ein stiftförmiges Stellmittel ausüben.

Die Zugangsausnehmung ist vorzugsweise derart ausgebildet und an einem Schenkel des Gabelkopfs angeordnet, dass ein stiftförmiges Stellmittel eines den Gabelkopf ergreifenden Handhabungsinstruments in die axiale Zugangsausnehmung eingreifen und den Aufnahmebereich des Druckstücks in axialer Richtung kraftbeaufschlagen kann.

Es kann sich aber auch als besonders vorteilhaft erweisen, wenn die Zugangsausnehmung ein Innengewinde trägt, in welche ein ein Außengewinde tragendes stiftförmiges Stellmittel einschraubbar ist und mit seinem freien Ende den Aufnahmebereich des Druckstücks in axialer Richtung kraftbeaufschlagen kann. Hierdurch lässt sich die temporäre Klemmkraft unabhängig von einem den Gabelkopf ergreifenden Handhabungsinstrument ausüben. Daher erweist es sich als besonders vorteilhaft, wenn das stiftförmige Stellmittel als ein von einem den Gabelkopf ergreifenden Handhabungsinstrument separates Bauteil ausgebildet ist und eine Werkzeugansetzstelle aufweist. Dies ermöglicht nämlich die temporäre Fixierung ohne dass zugleich ein Handhabungsinstrument an dem Gabelkopf fixiert werden muss. Dies erweist sich insbesondere bei komplexen chirurgischen Eingriffen, wie bei der Behandlung von Deformitäten, wie bei Skoliosen, als vorteilhaft, wo regelmäßig eine Vielzahl von Pedikelschrauben in engem Abstand zueinander gesetzt werden müssen. Hier würde eine Vielzahl von aus dem Patienten nach außen vorstehenden Handhabungsinstrumenten bei der chirurgischen Versorgung einer bestimmten Stelle stören. Daher erweist es sich als vorteilhaft, wenn das Handhabungsinstrument für den Gabelkopf diskonnektiert werden kann und dennoch mittels des stiftförmigen Stellmittels die intendierte Schwenkstellung des Gabelkopfs weiter temporär fixiert bleiben kann, was bei anderen bekannten Pedikelschrauben nach diesseitiger Kenntnis noch nicht realisiert wurde.

Die Erfindung erweist sich auch in Verbindung mit sogenannten Langkopfschrauben als vorteilhaft, bei denen die beiden Schenkel des Gabelkopfs jeweils einen bei einer Sollbruchstelle abtrennbaren distalen Schenkelabschnitt aufweisen, mittels derer der Gabelkopf in der axialen Richtung gewissermaßen verlängert ist. Bei der Implementierung der Erfindung auf solche Langkopfschrauben erweist es sich als vorteilhaft, wenn ein distaler Schenkelabschnitt eine mit der axialen Zugangsausnehmung fluchtende zweite Zugangsausnehmung aufweist, durch die das stiftförmige Stellmittel hindurchgreifen kann. In Weiterbildung dieses Gedankens erweist es sich als vorteilhaft, wenn beide miteinander fluchtenden Zugangsausnehmungen ein Innengewinde tragen und insbesondere weiter das stiftförmige Stellmittel zwei in axialer Richtung voneinander beabstandete Außengewindeabschnitte aufweist, die nach einer weiteren bevorzugten Ausführungsform einen geringeren Abstand voneinander aufweisen als die Innengewinde der axialen Zugangsausnehmungen. Der proximal angeordnete Außengewindeabschnitt des stiftförmigen Stellmittels hat dann die Aufgabe, das Stellmittel im nicht aktiven Zustand, also nach Lösen der temporären Klemmung, an dem distalen Schenkelabschnitt durch Einschrauben in das distale Innengewinde zu fixieren. Nach Ausführen aller chirurgischen Fixierungsmaßnahmen kann dann der betreffende Schenkel mit dem daran gehaltenen stiftförmigen Stellmittel an der Sollbruchlinie vom verbleibenden Gabelkopf abgetrennt werden.

Nach einer weiteren Ausführungsform erweist es sich als vorteilhaft, wenn das Innengewinde bei dem abtrennbaren Schenkelabschnitt eine größere lichte Durchgangsöffnung begrenzt als ein Außendurchmesser bei dem distalen Außengewindeabschnitt des stiftförmigen Stellmittels, so dass das stiftförmige Stellmittel mit seinem distalen Außengewindeabschnitt durch das Innengewinde bei dem abtrennbaren Schenkelabschnitt axial translatorisch hindurchsteckbar ist.

Es erweist sich des Weiteren als ganz besonders vorteilhaft, dass derjenige Schenkel des Gabelkopfs, bei welchem die Zugangsausnehmung ausgebildet ist, gegenüber dem anderen Schenkel in radialer Richtung verdickt ausgebildet ist. Hierdurch wird einerseits Material zur Ausbildung und Begrenzung der Zugangsausnehmung bei dem Schenkel bereitgestellt; andererseits wird hierdurch auch die Stabilität des Gabelkopfs insgesamt erhöht.

Wie eingangs erwähnt und wie bei der nicht vorveröffentlichten Druckschrift ebenfalls beschrieben, erweist es sich als vorteilhaft, wenn das Auflager zur schwenkbaren Anordnung des Druckelements ein in den Gabelkopf eingesetztes Stiftelement oder Wellenelement aufweist, das vorzugsweise in einer Ebene orthogonal zur axialen Richtung des Gabelkopfs erstreckt und in den Gabelkopf eingesetzt ist und gegen welches das Druckstück in axialer Richtung abgestützt ist.

Nach einer bevorzugten Ausführungsform ist das Auflager von einem freien Ende eines von außen nach radial innen durch einen Schenkel des Gabelkopfs gesteckten Stiftelements gebildet. Das Stiftelement wird zweckmäßigerweise erst nach dem Einsetzen und Einschwenken des Druckstücks in seine Montageposition am Gabelkopf montiert. Es wäre denkbar, dass sich das Druckstück lediglich von unten, also gegen eine distale Seite des Auflagers, abstützt. Auch hierdurch lässt sich erreichen, dass das Druckstück gewissermaßen als Klemmhebel fungiert. Die in axialer Richtung auf den Aufnahmebereich des Druckstücks wirkende temporäre Stellkraft wird nach den Hebelgesetzen verstärkt, etwa wie bei einem zwei Klammerschenkel aufweisenden Nussknacker. Hierbei stützt sich das Druckstück von unten an dem Auflager ab, also gegen die distale Seite des Auflagers, und drückt von oben auf den Kopf des Schraubankers.

Weiter kann es sich als vorteilhaft erweisen, wenn das genannte Stiftelement, welches das Auflager bildet, mit seinem freien Ende in eine demgegenüber größere radiale Öffnung des Druckstücks zumindest in der axialen Richtung spielbehaftet eingreift. Hierdurch ist das Druckstück bezüglich aller Richtungen verliersicher im Gabelkopf gehalten; es kann insbesondere schon vor der Montage des Schraubankers im Gabelkopf angebracht sein. Auf der anderen Seite bewirkt die Spielbehaftung in der axialen Richtung, dass sich das Druckstück bei der schlussendlichen dauerhaften Fixierung über ein weiteres Stellmittel, etwa eine in den Gabelkopf eingeschraubte Madenschraube, von seinem axialen Kontakt mit dem Auflager lösen kann und sich unter der Wirkung der schlussendlichen Klemmkraft optimal gegen den Kopf des Schraubankers abstützen kann.

Hierfür erweist es sich insbesondere auch als vorteilhaft, wenn das Druckstück im Bereich des Auflagers und relativ zu dem Auflager ein axiales Spiel aufweist, derart dass das Druckstück bei Krafteinleitung in axialer Richtung über das Korrekturelement eine axiale Stellbewegung ausführen kann, ohne dass diese Stellbewegung durch das Auflager behindert würde.

Weiter erweist es sich als vorteilhaft, wenn eine Klemmkraft zwischen dem Druckstück und dem Kopf des Schraubankers, die über Krafteinleitung auf den Aufnahmebereich des Druckstücks erzeugt wird, kleiner ist als eine Klemmkraft, die über Krafteinleitung mittels des weiteren Stellelements erzeugt wird. Solchenfalls kann nämlich das Druckstück bei der schlussendlichen dauerhaften Fixierung durch das weitere Stellelement seine optimale Stellung gegenüber dem Kopf des Schraubankers einnehmen.

Weitere Merkmale, Vorteile und Einzelheiten der Erfindung ergeben sich aus den beigefügten Patentansprüchen und der zeichnerischen Darstellung und nachfolgenden Beschreibung bevorzugter Ausführungsformen der erfindungsgemäßen Polyaxialschraube. In der Zeichnung zeigt:
Figuren 1 a-f verschiedene Ansichten einer erfindungsgemäßen Ausführungsform einer Polyaxialschraube und deren Bestandteilen;
Figuren 2 a-e verschiedene Ansichten des Druckstücks der Polyaxialschraube nach Figuren 1;
Figuren 3 a-e verschiedene Ansichten einer weiteren Ausführungsform einer erfindungsgemäßen Polyaxialschraube und deren Bestandteilen einschließlich eines Stift förmigen stellen mittels zur Ausübung einer temporären Klemmung zwischen Gabelkopf und Schraubankers zwischen Gabelkopf und Schraubanker.

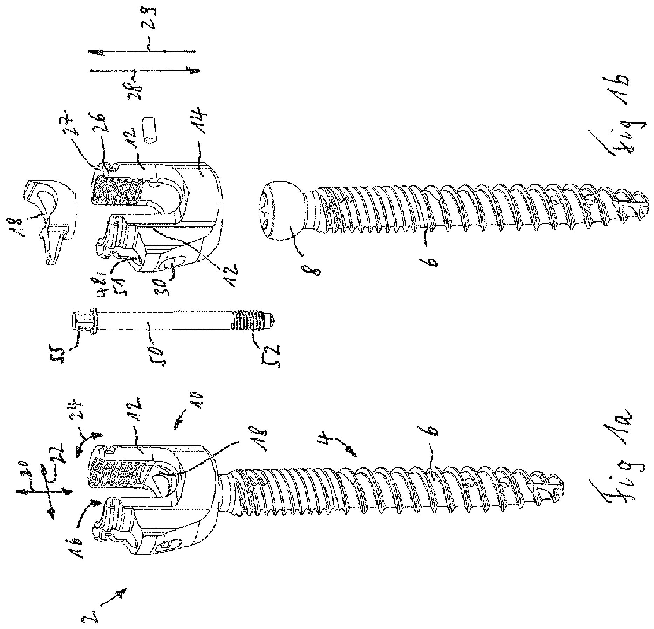

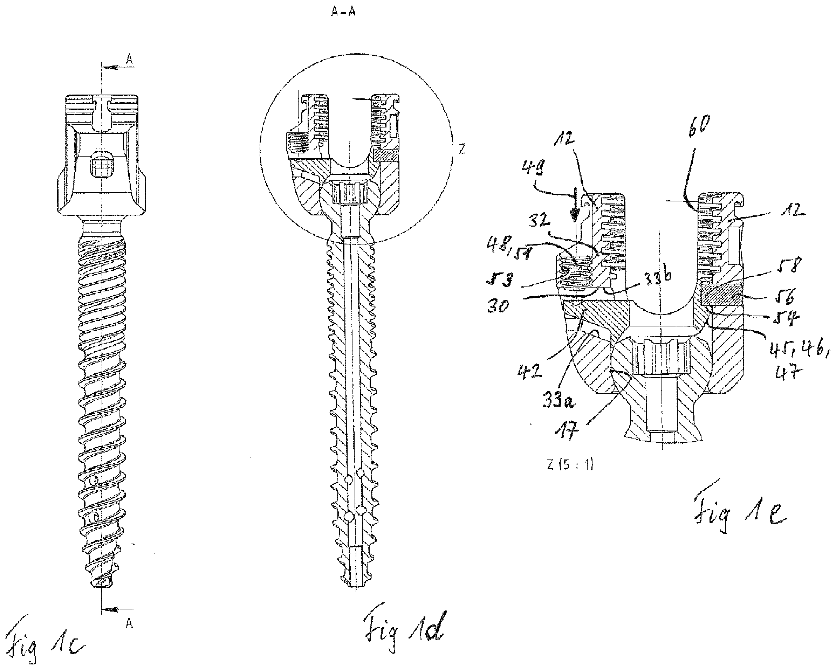

Die Figuren 1a-f zeigen eine erfindungsgemäße Polyaxialschraube 2 in Form einer Pedikelschraube. Die Pedikelschraube umfasst einen Schraubanker 4 mit Gewindeschaft 6 und Kopf 8 sowie einen Gabelkopf 10 mit zwei Schenkeln 12, die sich ausgehend von einem distalen Endbereich 14 des Gabelkopfs 10 in proximaler Richtung, also von dem Schraubanker 4 weg erstrecken und frei enden. Sie begrenzen eine U-förmige Aufnahmeöffnung 16 für ein Druckstück 18 und ein nicht dargestelltes Korrekturelement, insbesondere einen Korrekturstab, das in an sich bekannter Weise in die Aufnahmeöffnung 16 eingelegt wird und benachbarte oder gegebenenfalls mehrere Pedikelschrauben miteinander verbindet. Der Gabelkopf 10 weist an dem distalen Endbereich 14 eine in der Umfangsrichtung durchgehend begrenzte Durchstecköffnung 17 auf, so dass der Schraubanker 2 in seiner Längsrichtung durch diese Durchgangsöffnung 17 hindurch in den Gabelkopf 10 einsetzbar ist. Im eingesetzten Zustand ist der Kopf 8 des Schraubankers 4 in dem distalen Endbereich 14 des Gabelkopfs 10 polyaxial verschwenkbar gelagert. Bei Ausübung einer hinreichenden Kraft auf das Druckstück 18 in Richtung auf den Kopf 8 kann der Gabelkopf 10 gegenüber dem Kopf 8 des Schraubankers unverstellbar gestellt werden infolge der hierdurch erzeugten Klemmwirkung, was nachfolgend noch im Einzelnen erläutert werden wird. Der Gabelkopf 10 definiert des Weiteren eine axiale Richtung 20, eine hierzu radiale Richtung 22 sowie eine Umfangsrichtung 24, ferner den distalen Endbereich 14 und einen proximalen Endbereich 26 bzw. ein proximales Ende 27. Somit ist auch eine distale Richtung 28 und eine proximale Richtung 29 gegeben.

Ein Schenkel 12 des Gabelkopfs 10 weist auf seiner Innenseite eine Ausnehmung 30 auf, die sich ungefähr orthogonal zur axialen Richtung 20 in radialer Richtung 22 nach außen erstreckt. Im beispielhaft dargestellten Fall erstreckt sich die Ausnehmung 30 durch einen Wandbereich 32 des Schenkels 12 hindurch und mündet im Außenumfang des Schenkels 12. Sie ist nach radial außen verjüngt ausgebildet, indem sie von einer distalen Flanke 33a begrenzt ist, die von innen nach außen in proximaler Richtung 29 geneigt ist. Eine proximale Flanke 33b verläuft orthogonal zur axialen Richtung 20.

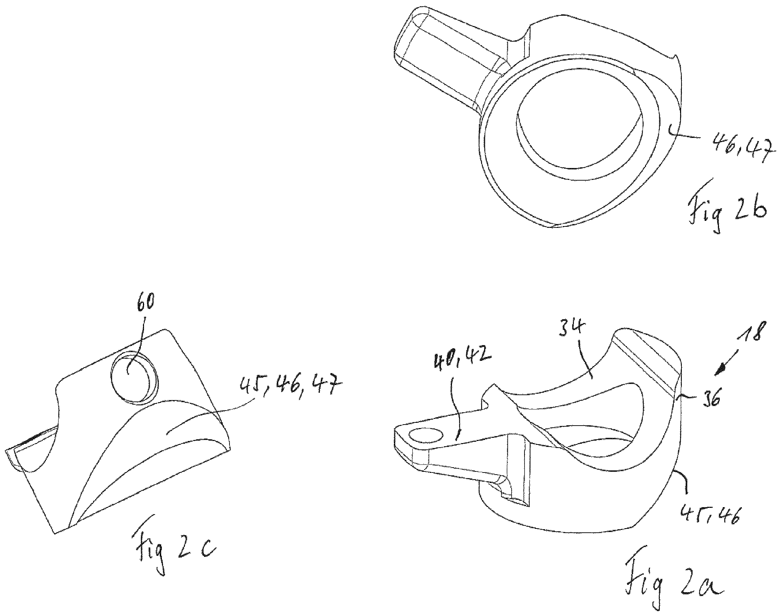

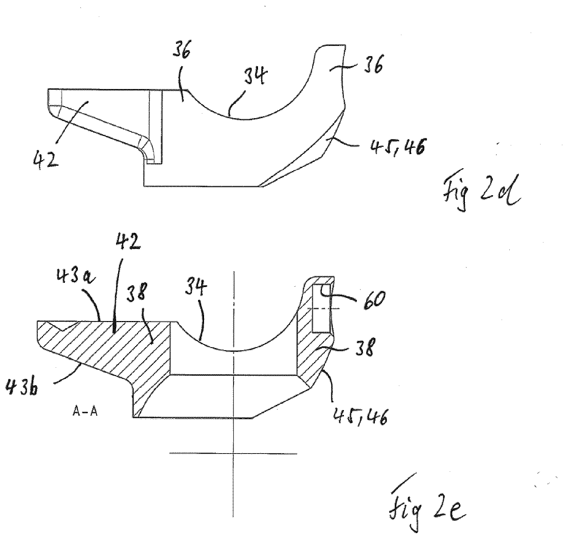

Wie man aus der Darstellung des Druckstücks 18 in den Figuren 2a-e ersehen kann, umfasst das Druckstück 18 eine ungefähr halbschalenförmige Aufnahme 34 für ein stabförmiges Korrekturelement. Diese halbschalenförmige Aufnahme 34 wird seitlich begrenzt durch in proximaler Richtung erstreckte Wandabschnitte 36, die einander gegenüberliegen und sich ausgehend von einem ringförmigen geschlossenen Basisabschnitt 38 des Druckstücks 18 in proximaler Richtung erstrecken. Ausgehend von einem der Wandabschnitte 36 erstreckt sich ein Ansatz oder Fortsatz 40 in radialer Richtung 22 nach außen. Dieser Ansatz oder Fortsatz 40 bildet einen Aufnahmebereich 42 für eine in axialer Richtung 20 wirkende Stellkraft, die auf noch zu beschreibende Weise auf das Druckstück 18 ausgeübt wird, um eine temporäre Klemmung zwischen dem Gabelkopf 10 und dem Schraubanker 4 zu realisieren. Der Aufnahmebereich 42 weist proximal eine orthogonal zur axialen Richtung 22 verlaufende Flanke 43a und distal eine schräg zur axialen Richtung 22 geneigte Flanke 43b auf.

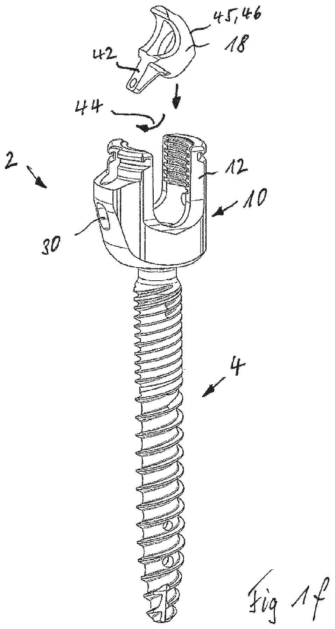

Ausgehend von der in Figur 1f dargestellten zur axialen Richtung 20 schrägen Orientierung des Druckstücks 18 lässt sich das Druckstück 18 in axialer Richtung 20 in den Gabelkopf 10 einführen und dann in die in den übrigen Figuren dargestellte Montagestellung in Richtung des angedeuteten Pfeils 44 einschwenken, wobei der Aufnahmebereich 42 zunehmend in die vorerwähnte Ausnehmung 30 eingreift. Dieses axiale Fügen des Druckstücks 18 in schräg geneigtem Zustand mit seinem Aufnahmebereich 42 voraus und das schlussendliche Einschwenken des Druckstücks 18 in seine Montageposition ist möglich, weil das Druckstücks 18 auf der Seite, welche dem Aufnahmebereich 42 gegenüberliegt, in einem distalen und dem Kopf 8 des Schraubankers 2 zugewandten Endbereich 45 eine Freischneidung 46 aufweist, die so ausgebildet ist, dass der distale Endbereich 45 beim axialen Einführen und Einschwenken des Druckstücks 18 nicht mit der Innenkontur des Gabelkopfs 10 und der Schenkel 12 kollidiert und hierdurch blockiert wird. Wie eingangs erwähnt dient die Freischneidung 46 genau diesem Zweck. Da der Gabelkopf 10 innen typischerweise konzentrisch ausgebildet sein kann, ist es hinreichend, wenn die Freischneidung von einer nach außen konvex gewölbten Fläche 47 begrenzt ist. Die Freischneidung könnte aber auch von insbesondere mehreren insbesondere ebenen facettenartig angeordneten Flächensegmenten begrenzt sein.

Wie in Figuren 1a, b-e dargestellt ragt der Aufnahmebereich 42 des Druckstücks 18 in die nach radial außen öffnende Ausnehmung 30 ein, so dass ein freies Ende des Aufnahmebereichs 42 zur Aufnahme einer in axialer Richtung wirkenden Stellkraft exponiert ist, die in Figur 1e mit einem Pfeil 49 angedeutet ist.

Man erkennt ferner, dass der betreffende Schenkel 12 des Gabelkopfs 10 eine in axialer Richtung 20 erstreckte Zugangsausnehmung 48 aufweist, die einen Zugang zu der nach radial außen öffnenden Ausnehmung 30 gestattet, in der der Aufnahmebereich 42 des Druckstücks 18 zu liegen kommt. Die Zugangsausnehmung 48 ist als in Umfangsrichtung durchgehend begrenzte axiale Öffnung 51 ausgebildet. Somit kann durch diese Zugangsausnehmung 48 hindurch mittels eines stiftförmigen Stellmittels eines die Pedikelschraube haltenden Handhabungsinstruments oder wie dargestellt eines anderen Stellmittels 50 (Figur 1b) eine Stellkraft in Richtung des Pfeils 49 in Figur 1e auf den Aufnahmebereich 42 des Druckstücks 18 ausgeübt werden kann. Das Stellmittel 50 kann im einfachsten Fall als Stift oder Stößel ausgebildet sein, der translatorisch in der axialen Richtung 20 durch die Zugangsausnehmung 48 hindurchgreift und sich gegen den Aufnahmebereich 42 des Druckstücks axial abstützt. Das Stellmittel 50 kann aber auch einen Außengewindeabschnitt 52 aufweisen, mit dem er in ein Innengewinde 53 in der Zugangsausnehmung 48 einschraubbar ist, so dass die auf den Aufnahmeabschnitt 42 ausgeübte Stellkraft einstellbar ist. Solchenfalls weist das Stellmittel 50 vorzugsweise auch eine Werkzeugansetzstelle 55 auf.

Das Druckstück 18 ist auf seiner dem Ansatz oder Fortsatz 40, welcher den Aufnahmebereich 42 die Stellkraft bildet, diametral gegenüberliegenden Seite so ausgebildet, dass es sich an einem Auflager 54 an dem anderen Schenkel 12 des Gabelkopfs 10 axial abstützen kann, und zwar derart, dass das Druckstück 18 bei Einleitung einer Stellkraft in Richtung des Pfeils 49 in einer die axiale Richtung 20 einschließenden Ebene in Richtung auf den Kopf 8 des Schraubankers 4 verschwenkbar ist.

Dieses Auflager 54 ist beispielhaft dadurch realisiert, dass ein Stiftelement 56 durch eine dem Stiftelement entsprechende Öffnung in dem Schenkel des Gabelkopfs von außen nach radialinnen eingesetzt, insbesondere eingepresst ist, so dass das radial innere freie Ende 58 des Stiftelements 56 das Auflager 54 bildet. Hierfür greift das freie Ende 58 in eine demgegenüber größere radiale Öffnung 60 bei dem Druckstück 18 ein. Dieser Eingriff ist zumindest in der axialen Richtung 20 spielbehaftet, was auch aus Figur 1e ersichtlich ist. Hierfür ist die radiale Öffnung 60 in der axialen Richtung 20 langlochförmig oder oval ausgebildet. Bei Einleitung der temporären Stellkraft in Richtung des Pfeils 49 auf den Aufnahmebereich 42 stützt sich das Druckstück 18 innerhalb seiner Öffnung 60 von unten, also gegen die distale Seite des freien Endes 58 des Stiftelements 56 ab, wodurch das Druckstücks Kraft auf den Kopf acht des Schraubankers in der axialen Richtung 20 ausübt und so die temporäre Klemmung bewirkt. Nach Wegnahme der temporären Stellkraft in Richtung des Pfeils 49 oder auch während dieser temporären Klemmung kann ein weiteres Stellelement, insbesondere eine an sich bekannte nicht dargestellte Madenschraube, in ein Innengewinde 60 der Schenkel 12 des Gabelkopfs 10 eingeschraubt werden und mittels des zuvor eingelegten Korrekturstabs den finalen Klemmdruck auf das Druckstück 18 in der axialen Richtung 20 ausüben. Dabei kann sich das Druckstück aufgrund der zuvor erwähnten Spielbehaftung in der axialen Richtung von der Abstützung an dem Auflager 54 lösen und seine schlussendliche optimale Klemmposition gegenüber dem Kopf des Schraubankers einnehmen.

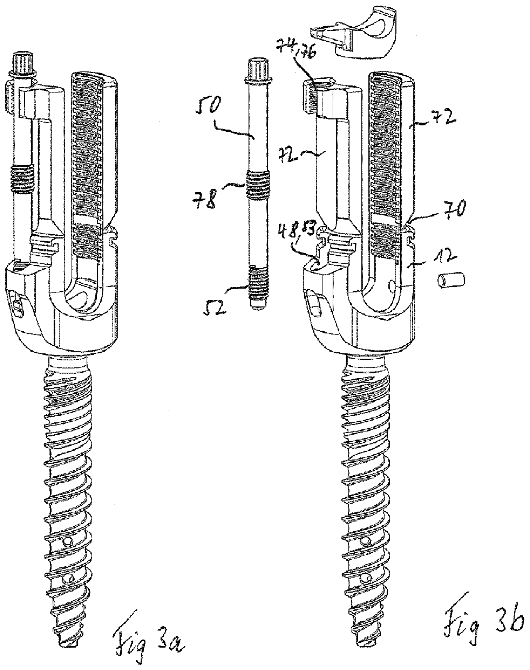

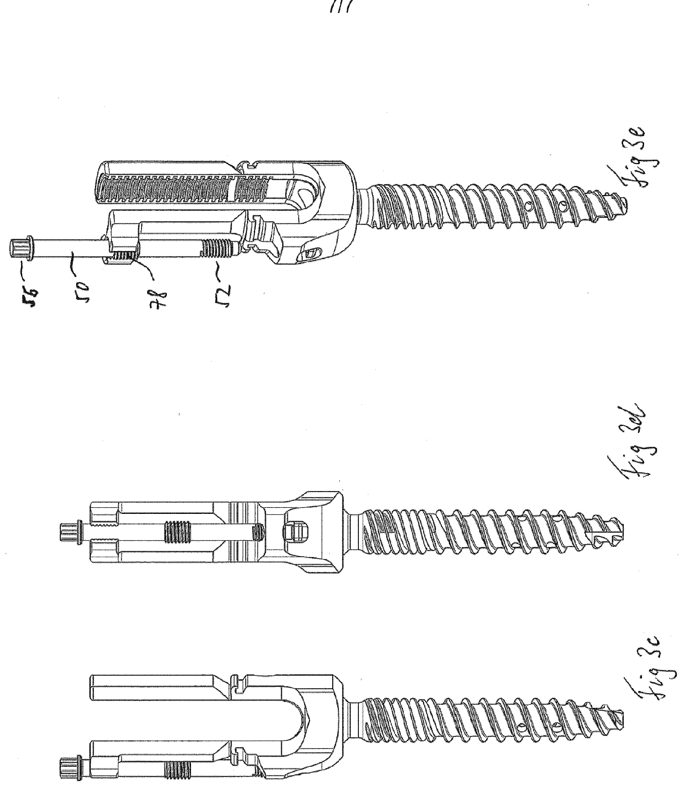

Die Figuren 3a-e zeigen eine weitere Ausführungsform der erfindungsgemäßen Polyaxialschraube, die hier als sogenannte Langschaftschraube ausgebildet ist. Die beiden Schenkel 12 weisen jeweils einen bei einer Sollbruchstelle 70 abtrennbaren distalen Schenkelabschnitt 72 auf. Einer der distalen Schenkelabschnitte 72 weist eine mit der axialen Zugangsausnehmung 48 fluchtende Öffnung 74 auf, so dass das stiftförmige Stellmittel 50 auch durch diese zweite Öffnung 74 hindurchgreifen kann. Vorteilhafterweise und beispielhaft weist hier sowohl die Zugangsausnehmung 48 als auch die zweite Öffnung 74 jeweils ein Innengewinde 53 bzw. 76 auf. Die zweite Öffnung 74 und deren Innengewinde 76 sind allerdings randoffen, also in axialer Richtung C-förmig ausgebildet, damit in radialer Richtung Bauraum eingespart wird. Auch das stiftförmige Stellmittel 50 weist einen distalen Außengewindeabschnitt 52 und einen weiteren proximalen Außengewindeabschnitt 78 auf. Das Innengewinde 76 hat einen geringfügig größeren Durchmesser als das Innengewinde 53, derart dass das Stift förmige Stellmittels 50 mit seinem distalen Außengewindeabschnitt 52 durch das Innengewinde 76 translatorisch hindurchgesteckt werden kann. Der Abstand der Außengewindeabschnitte 52, 78 voneinander ist aber vorzugsweise geringer als der Abstand der Innengewinde 53, 76 voneinander. Dies eröffnet die Möglichkeit, dass das stiftförmige Stellmittel in die in Figur 3e dargestellte zurückgezogene Position gebracht werden kann, in der der distale Außengewindeabschnitt 52 aus dem Innengewinde 53 der Zugangausnehmung 48 frei kommt, jedoch das stiftförmige Stellmittel 50 mit seinem proximalen Außengewindeabschnitt 78 in dem Innengewinde 76 des abtrennbaren Schenkelabschnitts 72 gehalten ist und zusammen mit diesem Schenkelabschnitt vom Gabelkopf abgetrennt werden kann.

## Patentansprüche

1. Polyaxialschraube (2), insbesondere Pedikelschraube, mit einem einen Gewindeschaft (6) und einen Kopf (8) aufweisenden Schraubanker (4) und mit einem in einer Seitenansicht U-förmigen und eine Aufnahmeöffnung (16) für ein Korrekturelement, insbesondere einen Korrekturstab, aufweisenden Gabelkopf (10) mit zwei Schenkeln (12), wobei der Gabelkopf (10) eine axiale Richtung (20), eine hierzu radiale Richtung (22) und eine Umfangsrichtung aufweist sowie ein distales Ende benachbart zu dem Schraubanker (4) und ein hiervon in axialer Richtung (20) abgewandtes proximales Ende (27) aufweist, so dass auch eine distale Richtung (28) und eine proximale Richtung (29) definiert ist,
wobei der Kopf (8) des Schraubankers (4) im eingesetzten Zustand in einem distalen Endbereich (14) des Gabelkopfs (10) polyaxial verschwenkbar gelagert ist und der Gabelkopf (10) in einer vom Chirurgen intendierten Schwenkstellung bezüglich des Kopfs (8) des im Knochen festgelegten oder festlegbaren Schraubankers (4) fixierbar ist,
wobei sich die Schenkel (12) ausgehend von einem distalen Bereich des Gabelkopfs (10) in proximaler Richtung (29) erstrecken und proximal frei enden und zwischen sich die Aufnahmeöffnung (16) für das Korrekturelement begrenzen,
wobei die Schenkel (12) einen radial äußeren Umfangsbereich aufweisen, in dem zum Ergreifen des Gabelkopfs (10) mittels eines Handhabungsinstruments wenigstens eine Haltenut oder eine sonstige Instrumentenansetzstelle ausgebildet ist,
und mit einem im Gabelkopf (10) zwischen dem Kopf (8) des Schraubankers (4) und dem Korrekturelement anordenbaren Druckstück (18), das einerseits auf dem Kopf (8) des Schraubankers (4) aufsitzt und andererseits durch das Korrekturelement belastbar ist,
wobei auf das Druckstück (18) mittels des am Gabelkopf (10) angreifenden Handhabungsinstruments oder mittels eines anderen Stellmittels eine temporär wirkende Kraft ausübbar ist, so dass der Gabelkopf (10) hierdurch gegenüber dem Kopf (8) des Schraubankers (4) temporär in einer vom Chirurgen gewünschten Schwenkstellung unverstellbar gehalten ist, solange bis der Gabelkopf und der Schraubanker durch ein anderes Stellmittel dauerhaft gegeneinander fixiert werden,
wobei das Druckstück (18) im Bereich eines Schenkels (12) des Gabelkopfs (10) über ein Auflager (54) gegen diesen Schenkel (12) in axialer Richtung (20) abgestützt ist und
wobei das Druckstück (18) diametral gegenüberliegend einen Aufnahmebereich (42) für eine in axialer Richtung (20) wirkende Stellkraft (49) aufweist, welche das Druckstück (18) in distaler Richtung bezüglich des Auflagers (54) zu verschwenken sucht und hierdurch die temporär wirkende Kraft in Richtung auf den Kopf (8) des Schraubankers (4) bewirkt,
wobei der Gabelkopf (10) eine in axialer Richtung (20) erstreckte Zugangsausnehmung (48) bei dem anderen Schenkel aufweist, durch welche Zugang zu dem Aufnahmebereich (42) des Druckstücks (18) mittels des Handhabungsinstruments oder des anderen Stellmittels (50) genommen werden kann, wobei dieser Schenkel des Gabelkopfs eine von innen in radialer Richtung (22) nach außen erstreckte Ausnehmung (30) aufweist, in welche das Druckstück (18) in seiner bestimmungsgemäßen Montageposition mit seinem Aufnahmebereich (42) von innen eingreift, **dadurch gekennzeichnet, dass** der Gabelkopf (10) im Bereich seines distalen Endes (14) eine in der Umfangsrichtung durchgehend begrenzte Durchstecköffnung (17) für den Schraubanker (2) aufweist, so dass der Schraubanker (2) in der axialen Richtung (20) durch diese Durchstecköffnung (17) hindurch in den Gabelkopf einsetzbar ist, und
dass das Druckstück (18) radial gegenüberliegend von seinem Aufnahmebereich (42) in einem distalen und dem Kopf des Schraubankers zugewandten Endbereich (45) eine derart ausgebildete randseitige Freischneidung (46) aufweist, dass das Druckstück (18) in schräg geneigtem Zustand mit seinem Aufnahmebereich (42) voraus in der axialen Richtung (20) von oben in den Gabelkopf (10) einsetzbar und in seine bestimmungsgemäße Montageposition einschwenkbar ist, in der das Druckstück (18) mit seinem Aufnahmebereich (42) in die nach radial außen erstreckte Ausnehmung (30) eingreift und auf dem Kopf (8) des Schraubankers (4) aufsitzt.

2. Polyaxialschraube nach Anspruch 1, **dadurch gekennzeichnet, dass** die beiden Schenkel (12) des Gabelkopfs einen insbesondere konzentrischen Innenraum begrenzen und dass das Druckstück (18) außerhalb seines Aufnahmebereichs (42) für die Stellkraft und außerhalb der Freischneidung (46) mit einem dem Innenraum entsprechenden insbesondere konzentrischen Umfang ausgebildet ist.

3. Polyaxialschraube nach Anspruch 1 oder 2, **dadurch gekennzeichnet, dass** die Freischneidung (46) von einer nach außen konvex gewölbten Fläche (47) oder von facettenartigen Flächensegmenten begrenzt ist.

4. Polyaxialschraube nach Anspruch 1, 2 oder 3, **dadurch gekennzeichnet, dass** die von innen nach radial außen erstreckte Ausnehmung (30) durch den Schenkel (12) des Gabelkopfs hindurch geht und am Außenumfang des Schenkels ausmündet, und sich insbesondere von radial innen nach radial außen verjüngt.

5. Polyaxialschraube nach einem oder mehreren der vorstehenden Ansprüche, **dadurch gekennzeichnet, dass** die von innen nach radial außen erstreckte Ausnehmung (30) von einer distalen Flanke (33a) begrenzt ist, die von innen nach außen in proximaler Richtung (29) geneigt ist.

6. Polyaxialschraube nach einem oder mehreren der vorstehenden Ansprüche, **dadurch gekennzeichnet, dass** der nach radial außen erstreckte Aufnahmebereich (42) des Druckstücks (18) radial innen in axialer Richtung dicker ausgebildet ist als radial weiter außen.

7. Polyaxialschraube nach einem oder mehreren der vorstehenden Ansprüche, **dadurch gekennzeichnet, dass** der nach radial außen erstreckte Aufnahmebereich (42) proximal eine orthogonal zur axialen Richtung (20) verlaufende Flanke (43a) und distal eine schräg zur axialen Richtung (20) geneigte Flanke (43b) aufweist.

8. Polyaxialschraube nach einem oder mehreren der vorstehenden Ansprüche, **dadurch gekennzeichnet, dass** die Zugangsausnehmung (48) zu dem Aufnahmebereich (42) des Druckstücks (18) als in ihrer Umfangsrichtung durchgehend begrenzte axiale Öffnung (51) ausgebildet ist.

9. Polyaxialschraube nach einem oder mehreren der vorstehenden Ansprüche, **dadurch gekennzeichnet, dass** die axiale Zugangsausnehmung (48) derart ausgebildet und an dem Schenkel des Gabelkopfs angeordnet ist, dass ein stiftförmiges Stellmittel eines den Gabelkopf ergreifenden Handhabungsinstruments in die axiale Zugangsausnehmung (48) eingreifen und den Aufnahmebereich (42) des Druckstücks (18) in axialer Richtung (20) kraftbeaufschlagen kann.

10. Polyaxialschraube nach einem oder mehreren der vorstehenden Ansprüche, **dadurch gekennzeichnet, dass** die axiale Zugangsausnehmung (48) ein Innengewinde (53) trägt, in welche ein einen Außengewindeabschnitt (52) tragendes stiftförmiges Stellmittel (50) einschraubbar ist und mit seinem freien Ende den Aufnahmebereich (42) des Druckstücks (18) in axialer Richtung (20) kraftbeaufschlagen kann.

11. Polyaxialschraube nach einem oder mehreren der vorstehenden Ansprüche, **dadurch gekennzeichnet, dass** der Gabelkopf (10) als Langkopf ausgebildet ist und die beiden Schenkel (12) einen bei einer Sollbruchstelle (70) abtrennbaren distalen Schenkelabschnitt (72) aufweisen, und dass ein distaler Schenkelabschnitt (72) eine mit der axialen Zugangsausnehmung (48) fluchtende zweite Öffnung (74) aufweist durch die ein stiftförmiges Stellmittel (50) hindurchgreifen kann.

12. Polyaxialschraube nach einem oder mehreren der vorstehenden Ansprüche, **dadurch gekennzeichnet, dass** derjenige Schenkel (12) des Gabelkopfs (10), bei welchem die Zugangsausnehmung (48) ausgebildet ist, gegenüber dem anderen Schenkel in radialer Richtung (22) verdickt ausgebildet ist.

13. Polyaxialschraube nach einem oder mehreren der vorstehenden Ansprüche, **dadurch gekennzeichnet, dass** das Auflager (54) ein in den Gabelkopf (10) eingesetztes Stiftelement (56) oder Wellenelement aufweist, das in einer Ebene orthogonal zur axialen Richtung (20) des Gabelkopfs (10) erstreckt und in den Gabelkopf eingesetzt ist und gegen welches das Druckstück (18) in axialer Richtung (20) abgestützt ist, und insbesondere das Auflager (54) von einem freien Ende (78) eines von außen nach radial innen durch einen Schenkel (12) des Gabelkopfs (10) gesteckten Stiftelements (56) gebildet ist.

14. Polyaxialschraube nach Anspruch 13, **dadurch gekennzeichnet, dass** das Stiftelement (56) mit seinem freien Ende (58) in eine demgegenüber größere radiale Öffnung (60) des Druckstücks (18) zumindest in der axialen Richtung (20) spielbehaftet eingreift.

15. Polyaxialschraube nach einem oder mehreren der vorstehenden Ansprüche, **dadurch gekennzeichnet, dass** das Druckstück (18) im Bereich des Auflagers (54) und relativ zu dem Auflager (54) ein axiales Spiel aufweist, derart dass das Druckstück (18) bei Krafteinleitung in axialer Richtung über das Korrekturelement eine axiale Stellbewegung ausführen kann, ohne dass diese Stellbewegung durch das Auflager (54) behindert würde.

## Claims

1. Polyaxial screw (2), in particular a pedicle screw, comprising a screw anchor (4) which has a threaded shaft (6) and a head (8), and comprising a fork head (10) which has two arms (12), is U-shaped in a lateral view and has a receiving opening (16) for a correction element, in particular a correction rod, the fork head (10) having an axial direction (20), a radial direction (22) in relation thereto and a peripheral direction, and having a distal end adjacent to the screw anchor (4) and a proximal end (27) facing away from said screw anchor in the axial direction (20), such that a distal direction (28) and a proximal direction (29) are also defined,
the head (8) of the screw anchor (4), when inserted, being mounted in a distal end region (14) of the fork head (10) so as to be polyaxially pivotable, and it being possible for the fork head (10) to be fixed in a pivot position, which is intended by the surgeon, with respect to the head (8) of the screw anchor (4) which is fixed or can be fixed in the bone,
the arms (12) extending in the proximal direction (29) proceeding from a distal region of the fork head (10), ending proximally freely, and between themselves delimiting the receiving opening (16) for the correction element,
the arms (12) having a radially outer peripheral region in which at least one holding groove or another instrument attachment point is formed in order to grip the fork head (10) by means of a handling instrument,
and the polyaxial screw comprising a pressure piece (18) which can be arranged in the fork head (10) between the head (8) of the screw anchor (4) and the correction element, rests on the head (8) of the screw anchor (4) and can be loaded by the correction element,
it being possible for a temporarily acting force to be exerted on the pressure piece (18) by means of the handling instrument acting on the fork head (10) or by means of another actuating element, such that, as a result, the fork head (10) is temporarily non-adjustably held in relation to the head (8) of the screw anchor (4) in a pivot position desired by the surgeon, until the fork head and the screw anchor are permanently fixed against one another by means of a further actuating element,
the pressure piece (18) being supported, in the region of an arm (12) of the fork head (10), against said arm (12) in the axial direction (20) by means of a supporting element (54),
and the pressure piece (18) having a receiving region (42) diametrically opposite for an actuating force (49) acting in the axial direction (20), which actuating force tries to pivot the pressure piece (18) in the distal direction with respect to the supporting element (54), thus effecting the temporarily acting force in the direction of the head (8) of the screw anchor (4),
the fork head (10) having an access hole (48) on the other arm, which access hole extends in the axial direction (20) and through which the receiving region (42) of the pressure piece (18) can be accessed by the handling instrument or the other actuating element (50), said arm of the fork head having a hole (30) which extends from the inside in the radial direction (22) to the outside and into which the pressure piece (18) engages with its receiving region (42) from the inside when said pressure piece is in its intended mounting position, **characterized in that** the fork head (10), in the region of its distal end (14), has a through-opening (17) for the screw anchor (2), which through-opening is continuously delimited in the peripheral direction, such that the screw anchor (2) can be inserted into the fork head through said through-opening (17) in the axial direction (20), and
**in that** the pressure piece (18) has a cut-out (46) radially opposite from the receiving region (42) thereof, in a distal end region (45) which faces the head of the screw anchor, which cut-out is designed such that the pressure piece (18) with its receiving region (42) ahead can be inserted into the fork head (10) in the axial direction (20) from above when obliquely inclined, and pivoted into its intended mounting position, in which the pressure piece (18) with its receiving region (42) engages into the hole (30), which extends radially outward, and rests on the head (8) of the screw anchor (4) .

2. Polyaxial screw according to claim 1, **characterized in that** the two arms (12) of the fork head delimit an in particular concentric interior, and **in that** the pressure piece (18) is designed to have an in particular concentric periphery corresponding to the interior, except for its receiving region (42) for the actuating force and except for the cut-out (46).

3. Polyaxial screw according to claim 1 or claim 2, **characterized in that** the cut-out (46) is delimited by an outwardly convex surface (47) or by faceted surface segments.

4. Polyaxial screw according to claim 1, claim 2 or claim 3, **characterized in that** the hole (30) which extends from the inside to the radial outside passes through the arm (12) of the fork head and opens out at the outer periphery of the arm, and in particular tapers from the radial inside to the radial outside.

5. Polyaxial screw according to one or more of the preceding claims, **characterized in that** the hole (30) which extends from the inside to the radial outside is delimited by a distal flank (33a) which is inclined from the inside to the outside in the proximal direction (29).

6. Polyaxial screw according to one or more of the preceding claims, **characterized in that** the receiving region (42) of the pressure piece (18), which receiving region extends to the radial outside, is thicker on the radial inside in the axial direction than radially further out.

7. Polyaxial screw according to one or more of the preceding claims, **characterized in that** the receiving region (42) which extends to the radial outside proximally has a flank (43a) which extends orthogonally to the axial direction (20) and distally has a flank (43b) which is obliquely inclined to the axial direction (20).

8. Polyaxial screw according to one or more of the preceding claims, **characterized in that** the access hole (48) to the receiving region (42) of the pressure piece (18) is designed as an axial opening (51) which is continuously delimited in the peripheral direction thereof.

9. Polyaxial screw according to one or more of the preceding claims, **characterized in that** the axial access hole (48) is designed and arranged on the arm of the fork head, such that a pin-shaped actuating element of a handling instrument gripping the fork head can engage in the axial access hole (48) and can apply force to the receiving region (42) of the pressure piece (18) in the axial direction (20).

10. Polyaxial screw according to one or more of the preceding claims, **characterized in that** the axial access hole (48) carries an internal thread (53) into which a pin-shaped actuating element (50) carrying an external thread portion (52) can be screwed and of which the free end can apply force to the receiving region (42) of the pressure piece (18) in the axial direction (20).

11. Polyaxial screw according to one or more of the preceding claims, **characterized in that** the fork head (10) is designed as an elongate head and the two arms (12) have a distal arm portion (72) which can be separated at a predetermined breaking point (70), and **in that** a distal arm portion (72) has a second opening (74) which is aligned with the axial access hole (48), through which opening a pin-shaped actuating element (50) can pass.

12. Polyaxial screw according to one or more of the preceding claims, **characterized in that** the particular arm (12) of the fork head (10), in which the access hole (48) is formed, is thickened in the radial direction (22) in relation to the other arm.

13. Polyaxial screw according to one or more of the preceding claims, **characterized in that** the supporting element (54) has a pin element (56) or shaft element which is inserted into the fork head (10), extends in a plane orthogonally to the axial direction (20) of the fork head (10) and is inserted into the fork head and against which the pressure piece (18) is supported in the axial direction (20), in particular wherein the supporting element (54) is formed by a free end (58) of a pin element (56) which is inserted from the outside to the radial inside by an arm (12) of the fork head (10).

14. Polyaxial screw according to claim 13, **characterized in that** the pin element (56) with its free end (58) engages with play in a relatively larger radial opening (60) of the pressure piece (18) at least in the axial direction (20) .

15. Polyaxial screw according to one or more of the preceding claims, **characterized in that** the pressure piece (18) has an axial play in the region of the supporting element (54) and relative to the supporting element (54), such that the pressure piece (18), when force is applied in the axial direction, can carry out an axial actuation movement by means of the correction element without said actuation movement being impeded by the supporting element (54).

## Revendications

1. Vis polyaxiale (2), en particulier vis pédiculaire, avec un ancrage à visser (4) présentant une tige filetée (6) et une tête (8) et avec une tête en fourche (10) en forme de U dans une vue latérale et présentant une ouverture de réception (16) pour un élément de correction, en particulier une barre de correction, avec deux branches (12), dans laquelle la tête en fourche (10) présente une direction axiale (20), une direction radiale (22) par rapport à celle-ci et une direction périphérique ainsi que présente une extrémité distale au voisinage de l'ancrage à visser (4) et une extrémité proximale (27) opposée à celle-ci dans la direction axiale (20), de sorte qu'une direction distale (28) et une direction proximale (29) est également définie,
dans laquelle la tête (8) de l'ancrage à visser (4) dans l'état inséré dans une zone d'extrémité distale (14) de la tête en fourche (10) est montée de manière à pouvoir pivoter de façon polyaxiale et la tête en fourche (10) peut être fixée dans une position de pivotement visée par le chirurgien par rapport à la tête (8) de l'ancrage à visser (4) immobilisé ou pouvant être immobilisé dans l'os,
dans laquelle les branches (12) s'étendent à partir d'une zone distale de la tête en fourche (10) dans la direction proximale (29) et terminent librement de manière proximale et délimitent entre elles l'ouverture de réception (16) pour l'élément de correction,
dans laquelle les branches (12) présentent une zone périphérique radialement extérieure, dans laquelle au moins une rainure de retenue ou un autre emplacement d'application d'instrument est réalisé(e) pour la saisie de la tête en fourche (10) au moyen d'un instrument de manipulation,
et avec une pièce de pression (18) pouvant être disposée dans la tête en fourche (10) entre la tête (8) de l'ancrage à visser (4) et l'élément de correction, qui d'une part repose sur la tête (8) de l'ancrage à visser (4) et d'autre part peut être sollicitée par l'élément de correction,
dans laquelle une force agissant temporairement peut être exercée sur la pièce de pression (18) au moyen de l'instrument de manipulation en contact avec la tête en fourche (10) ou au moyen d'un autre moyen de réglage, de sorte que la tête en fourche (10) est ainsi retenue de manière immobile par rapport à la tête (8) de l'ancrage à visser (4) temporairement dans une position de pivotement souhaitée par le chirurgien, jusqu'à ce que la tête en fourche et l'ancrage à visser soient fixés l'un par rapport à l'autre durablement par un autre moyen de réglage,
dans laquelle la pièce de pression (18) est en appui dans la zone d'une branche (12) de la tête en fourche (10) dans la direction axiale (20) par l'intermédiaire d'un support (54) contre cette branche (12) et
dans laquelle la pièce de pression (18) présente de manière diamétralement opposée une zone de réception (42) pour une force de réglage (49) agissant dans la direction axiale (20), laquelle cherche à faire pivoter la pièce de pression (18) dans la direction distale par rapport au support (54) et provoque ainsi la force agissant temporairement en direction de la tête (8) de l'ancrage à visser (4),
dans laquelle la tête en fourche (10) présente un évidement d'accès (48) étendu dans la direction axiale (20) pour l'autre branche, par lequel un accès à la zone de réception (42) de la pièce de pression (18) au moyen de l'instrument de manipulation ou de l'autre moyen de réglage (50) peut être donné, dans laquelle cette branche de la tête en fourche présente un évidement (30) étendu à partir de l'intérieur dans la direction radiale (22) vers l'extérieur, dans lequel la pièce de pression (18) s'insère de l'intérieur dans sa position de montage conforme à l'usage prévu avec sa zone de réception (42), **caractérisée en ce que** la tête en fourche (10) présente dans la zone de son extrémité distale (14) une ouverture de passage (17) délimitée en continu dans la direction périphérique pour l'ancrage à visser (2), de sorte que l'ancrage à visser (2) peut être inséré dans la direction axiale (20) à travers cette ouverture de passage (17) dans la tête en fourche, et
que la pièce de pression (18) présente radialement à l'opposé de sa zone de réception (42) dans une zone d'extrémité (45) distale et tournée vers la tête de l'ancrage à visser un dégagement (46) côté bord réalisé de telle sorte que la pièce de pression (18) peut être insérée dans l'état incliné de manière oblique avec sa zone de réception (42) vers l'avant dans la direction axiale (20) par le dessus dans la tête en fourche (10) et peut entrer en pivotant dans sa position de montage conforme à l'usage prévu, dans laquelle la pièce de pression (18) s'insère avec sa zone de réception (42) dans l'évidement (30) étendu vers radialement à l'extérieur et repose sur la tête (8) de l'ancrage à visser (4).

2. Vis polyaxiale selon la revendication 1, **caractérisée en ce que** les deux branches (12) de la tête en fourche délimitent un espace intérieur en particulier concentrique et que la pièce de pression (18) est réalisée à l'extérieur de sa zone de réception (42) pour la force de réglage et à l'extérieur du dégagement (46) avec une périphérie en particulier concentrique correspondant à l'espace intérieur.

3. Vis polyaxiale selon la revendication 1 ou 2, **caractérisée en ce que** le dégagement (46) est délimité par une surface (47) bombée de manière convexe vers l'extérieur ou des segments de surface du type facettes.

4. Vis polyaxiale selon la revendication 1, 2 ou 3, **caractérisée en ce que** l'évidement (30) étendu de l'intérieur vers radialement à l'extérieur traverse la branche (12) de la tête en fourche et débouche sur la périphérie extérieure de la branche, et s'amincit en particulier à partir de radialement à l'intérieur vers radialement à l'extérieur.

5. Vis polyaxiale selon l'une ou plusieurs des revendications précédentes, **caractérisée en ce que** l'évidement (30) étendu de l'intérieur vers radialement à l'extérieur est délimité par un flanc distal (33a), qui est incliné de l'intérieur vers l'extérieur dans la direction proximale (29).

6. Vis polyaxiale selon l'une ou plusieurs des revendications précédentes, **caractérisée en ce que** la zone de réception (42) étendue vers radialement à l'extérieur de la pièce de pression (18) est réalisée radialement à l'intérieur dans la direction axiale de manière plus épaisse que radialement plus à l'extérieur.

7. Vis polyaxiale selon l'une ou plusieurs des revendications précédentes, **caractérisée en ce que** la zone de réception (42) étendue vers radialement à l'extérieur présente de manière proximale un flanc (43a) s'étendant perpendiculairement par rapport à la direction axiale (20) et de manière distale un flanc (43b) incliné de manière oblique par rapport à la direction axiale (20) .

8. Vis polyaxiale selon l'une ou plusieurs des revendications précédentes, **caractérisée en ce que** l'évidement d'accès (48) menant à la zone de réception (42) de la pièce de pression (18) est réalisé sous la forme d'une ouverture axiale (51) délimitée en continu dans sa direction périphérique.

9. Vis polyaxiale selon l'une ou plusieurs des revendications précédentes, **caractérisée en ce que** l'évidement d'accès (48) axial est réalisé et disposé sur la branche de la tête en fourche de telle sorte qu'un moyen de réglage en forme de tige d'un instrument de manipulation saisissant la tête en fourche peut s'insérer dans l'évidement d'accès (48) axial et soumettre la zone de réception (42) de la pièce de pression (18) dans la direction axiale (20) à une force.

10. Vis polyaxiale selon l'une ou plusieurs des revendications précédentes, **caractérisée en ce que** l'évidement d'accès (48) axial porte un filetage femelle (53), dans lequel un moyen de réglage (50) en forme de tige portant une partie de filetage mâle (52) peut être vissé et avec son extrémité libre peut soumettre la zone de réception (42) de la pièce de pression (18) dans la direction axiale (20) à une force.

11. Vis polyaxiale selon l'une ou plusieurs des revendications précédentes, **caractérisée en ce que** la tête en fourche (10) est réalisée sous la forme d'une tête longitudinale et les deux branches (12) présentent une partie de branche (72) distale séparable en un point de rupture (70), et qu'une partie de branche distale (72) présente une deuxième ouverture (74) en affleurement avec l'évidement d'accès (48) axial, par laquelle un moyen de réglage (50) en forme de tige peut passer.

12. Vis polyaxiale selon l'une ou plusieurs des revendications précédentes, **caractérisée en ce que** la branche (12) de la tête en fourche (10), pour laquelle l'évidement d'accès (48) est réalisé, est réalisée de manière épaissie dans la direction radiale (22) par rapport à l'autre branche.

13. Vis polyaxiale selon l'une ou plusieurs des revendications précédentes, **caractérisée en ce que** le support (54) présente un élément tige (56) ou élément ondulé inséré dans la tête en fourche (10), qui s'étend dans un plan perpendiculairement par rapport à la direction axiale (20) de la tête en fourche (10) et est inséré dans la tête en fourche et contre lequel la pièce de pression (18) est en appui dans la direction axiale (20), et en particulier le support (54) est formé par une extrémité libre (78) d'un élément tige (56) enfoncé de l'extérieur vers radialement à l'intérieur à travers une branche (12) de la tête en fourche (10).

14. Vis polyaxiale selon la revendication 13, **caractérisée en ce que** l'élément tige (56) s'insère avec du jeu avec son extrémité libre (58) dans une ouverture radiale (60) de la pièce de pression (18) plus grande par rapport à celle-ci au moins dans la direction axiale (20).

15. Vis polyaxiale selon l'une ou plusieurs des revendications précédentes, **caractérisée en ce que** la pièce de pression (18) présente dans la zone du support (54) et par rapport au support (54) un jeu axial, de telle sorte que la pièce de pression (18) lors de l'introduction d'une force peut réaliser dans la direction axiale un mouvement de réglage axial par l'intermédiaire de l'élément de correction, sans que ce mouvement de réglage ne soit gêné par le support (54).
